# EUROPEAN PATENT SPECIFICATION

(11) **EP 3 628 243 B1**
(45) Date of publication and mention of the grant of the patent: **07.10.2026**
(21) Application number: 19209511.5
(22) Date of filing: 20.10.2015
(51) Int. Cl.: A61B 17/122, A61B 17/128, A61B 17/00, A61B 17/08

(54) **IMPROVED GRASPING FOR TISSUE REPAIR**
VERBESSERTE AUFNAHME FÜR GEWEBEREPARATUR
PRÉHENSION AMÉLIORÉE POUR LA RÉPARATION DE TISSUS

(30) Priority: 19.12.2014 US 201414577852
(43) Date of publication of application: 01.04.2020
(62) Document number(s) of the earlier application(s) in accordance with Art. 76 EPC: 15787422.3 / 3 232 948
(73) Proprietor: Abbott Cardiovascular Systems, Inc., Santa Clara, CA 95054 (US)
(72) Inventor: WEI, Michael F., Redwood City California 94065 (US)
(74) Representative: Boult Wade Tennant LLP

(56) References cited:
- WO-A2-2006/037073
- US-A1- 2004 049 207
- US-A1- 2007 038 293
- US-A1- 2010 022 823

## Description

### BACKGROUND

The present invention relates generally to medical devices. In particular, the present invention relates to devices for the endovascular, percutaneous, or minimally invasive surgical treatment of bodily tissues, such as tissue approximation or valve repair. More particularly, the present invention relates to repairing heart valves and venous valves, and devices for removing or disabling mitral valve repair components through minimally invasive procedures.

Surgical repair of bodily tissues often involves tissue approximation and fastening of such tissues in the approximated arrangement. When repairing valves, tissue approximation includes coapting the leaflets of the valves in a therapeutic arrangement which may then be maintained by fastening or fixing the leaflets. Such coaptation can be used to treat regurgitation which most commonly occurs in the mitral valve.

Mitral valve regurgitation is characterized by retrograde flow from the left ventricle of a heart through an incompetent mitral valve into the left atrium. During a normal cycle of heart contraction (systole), the mitral valve acts as a check valve to prevent oxygenated blood from flowing back into the left atrium. In this way, oxygenated blood is pumped into the aorta through the aortic valve. Mitral valve regurgitation can significantly decrease the pumping efficiency of the heart, placing the patient at risk of severe, progressive heart failure.

Mitral valve regurgitation can result from a number of different mechanical defects in the mitral valve or the left ventricular wall. The valve leaflets, the valve chordae which connect the leaflets to the papillary muscles, the papillary muscles themselves, or the left ventricular wall may be damaged or otherwise dysfunctional. Commonly, the valve annulus may be damaged, dilated, or weakened, limiting the ability of the mitral valve to close adequately against the high pressures of the left ventricle.

The most common treatments for mitral valve regurgitation rely on valve replacement or repair including leaflet and annulus remodeling, the latter generally referred to as valve annuloplasty. One technique for mitral valve repair which relies on suturing adjacent segments of the opposed valve leaflets together is referred to as the "bow-tie" or "edge-to-edge" technique. While all these techniques can be effective, they usually rely on open heart surgery where the patient's chest is opened, typically via a sternotomy, and the patient placed on cardiopulmonary bypass. The need to both open the chest and place the patient on bypass is traumatic and has associated high mortality and morbidity.

In some patients, a fixation device can be installed into the heart using minimally invasive techniques. The fixation device can hold the adjacent segments of the opposed valve leaflets together and may reduce mitral valve regurgitation. One such device used to clip the anterior and posterior leaflets of the mitral valve together is the MitraClip^{®} fixation device, sold by Abbott Vascular, Santa Clara, California, USA.

### DESCRIPTION OF THE BACKGROUND ART

Many techniques exist for approximating and repairing tissues and organs at treatment sites. For example, minimally invasive and percutaneous techniques for coapting and modifying mitral valve leaflets to treat mitral valve regurgitation are described in PCT Publication Nos. WO 98/35638; WO 99/00059; WO 99/01377; and WO 00/03759; WO 2000/060995; WO 2004/103162. Maisano et al. (1998) Eur. J. Cardiothorac. Surg. 13:240-246; Fucci et al. (1995) Eur. J. Cardiothorac. Surg. 9:621-627; and Umana et al. (1998) Ann. Thorac. Surg. 66:1640-1646, describe open surgical procedures for performing "edge-to-edge" or "bow-tie" mitral valve repair where edges of the opposed valve leaflets are sutured together to lessen regurgitation. Dec and Fuster (1994) N. Engl. J. Med. 331:1564-1575 and Alvarez et al. (1996) J. Thorac. Cardiovasc. Surg. 112:238-247 are review articles discussing the nature of and treatments for dilated cardiomyopathy.

Mitral valve annuloplasty is described in the following publications: Bach and Bolling (1996) Am. J. Cardiol. 78:966-969; Kameda et al. (1996) Ann. Thorac. Surg. 61:1829-1832; Bach and Bolling (1995) Am. Heart J. 129:1165-1170; and Bolling et al. (1995) 109:676-683. Linear segmental annuloplasty for mitral valve repair is described in Ricchi et al. (1997) Ann. Thorac. Surg. 63:1805-1806. Tricuspid valve annuloplasty is described in McCarthy and Cosgrove (1997) Ann. Thorac. Surg. 64:267-268; Tager et al. (1998) Am. J. Cardiol. 81:1013-1016; and Abe et al. (1989) Ann. Thorac. Surg. 48:670-676.

Percutaneous transluminal cardiac repair procedures are described in Park et al. (1978) Circulation 58:600-608; Uchida et al. (1991) Am. Heart J. 121: 1221-1224; and Ali Khan et al. (1991) Cathet. Cardiovasc. Diagn. 23:257-262. Endovascular cardiac valve replacement is described in U.S. Patent Nos. 5,840,081; 5,411,552; 5,554,185; 5,332,402; 4,994,077; and 4,056,854. U.S. Patent No. 3,671,979 describes a catheter for temporary placement of an artificial heart valve.

Other percutaneous and endovascular cardiac repair procedures are described in U.S. Patent Nos. 4,917,089; 4,484,579; and 3,874,338; and PCT Publication No. WO 91/01689.

Thoracoscopic and other minimally invasive heart valve repair and replacement procedures are described in U.S. Patent Nos. 5,855,614; 5,829,447; 5,823,956; 5,797,960; 5,769,812; and 5,718,725.

In WO 2006/037073 and US 2010/0022823 there are described devices, systems and methods for stabilizing and grasping tissues such as valve leaflets, assessing the grasp of these tissues, approximating and fixating the tissues, and assessing the fixation of the tissues to treat cardiac valve regurgitation, particularly mitral valve regurgitation.

In US 2004/0049207 there are described devices, systems and methods for tissue approximation and repair at treatment sites. The devices, systems and methods are said to find use in a variety of therapeutic procedures, including endovascular, minimally-invasive, and open surgical procedures, and can be used in various anatomical regions, including the abdomen, thorax, cardiovascular system, heart, intestinal tract, stomach, urinary tract, bladder, lung, and other organs, vessels, and tissues. The devices are said to be particularly useful in those procedures requiring minimally-invasive or endovascular access to remote tissue locations, where the instruments utilized must negotiate long, narrow, and tortuous pathways to the treatment site. In addition, many of the devices and systems are adapted to be reversible and removable from the patient at any point without interference with or trauma to internal tissues.

### BRIEF SUMMARY

The present disclosure describes devices intended for intravascular delivery and for use in treating mitral valve defects in human patients. The mitral valve of a human heart has an atrial side, a ventricular side, an anterior leaflet, a posterior leaflet, and an opening between the leaflets.

According to the present invention there is provided a device having the features of claim 1 configured for intravascular delivery and for use in treating mitral valve defects in human patients.

In one embodiment, the device includes a body, a pair of proximal elements, and a pair of distal elements. Each proximal element has a first end and a free second end. Each proximal element has a proximal engagement surface between its first and second ends. Each proximal engagement surface is configured to approximate and engage a portion of the leaflets adjacent the mitral valve on the atrial side.

Each distal element is pivotally coupled at a first end to the body on opposite sides of the body, and has a free second end. Each distal element has a distal engagement surface between its first and second ends. Each distal engagement surface is configured to approximate and engage a portion of the leaflets adjacent the mitral valve on the ventricular side.

A first one of the proximal elements cooperates with a first one of the distal elements to form a space for receiving a portion of the anterior leaflet therebetween. A second one of the proximal elements cooperates with a second one of the distal elements to form a space for receiving a portion of the posterior leaflet therebetween. Each such space has an open end and a closed end, and the closed end forms an apex.

The device includes an actuator for selectively moving the distal elements between a first position in which the distal elements are in a collapsed, low profile configuration for delivery of the device, a second position in which the distal elements are in an expanded configuration for positioning the device relative to the mitral valve, and a third position in which the distal elements are secured in position against a portion of the leaflets adjacent the mitral valve on the ventricular side.

The device also includes an actuator for selectively moving the proximal elements between a first position in which the proximal elements are in a collapsed, low profile configuration for delivery of the device and a second position in which the proximal elements are in an expanded configuration for engaging a portion of the leaflets adjacent the mitral valve on the atrial side.

Each distal element also includes a hinged or pivoting arm positioned along the distal engagement surface. Each hinged or pivoting arm is configured to cooperate with a corresponding frictional element to capture a free edge of the mitral valve leaflet as the device is positioned relative to the mitral valve. Each hinged or pivoting arm is configured to cooperate with a frictional element to allow a leading free edge of the leaflets to move in a first direction toward the body with little or no resistance or restriction and to resist or prevent movement of the free edge of the leaflets in an opposite direction away from the body.

These and other objects and features of the present disclosure will become more fully apparent from the following description and appended claims, or may be learned by the practice of the embodiments of the invention as set forth hereinafter.

### BRIEF DESCRIPTION OF THE DRAWINGS

To further clarify the above and other advantages and features of the present disclosure, a more particular description of the invention will be rendered by reference to specific embodiments thereof which are illustrated in the appended drawings. It is appreciated that these drawings depict only illustrated embodiments of the invention and are therefore not to be considered limiting of its scope. Embodiments of the invention will be described and explained with additional specificity and detail through the use of the accompanying drawings in which:
Figure 1 illustrates free edges of leaflets of the mitral valve in normal coaptation, and Figure 2 illustrates the free edges in regurgitative coaptation.
Figures 3A-3C illustrate grasping of the leaflets with a fixation device, inversion of the distal elements of the fixation device and removal of the fixation device, respectively.
Figure 4 illustrates the fixation device in a desired orientation relative to the leaflets.
Figure 5 illustrates an exemplary fixation device coupled to a shaft.
Figures 6A-6B, 7A-7B, and 8 illustrate a fixation device in various possible positions during introduction and placement of the device within the body to perform a therapeutic procedure.
Figures 9A-9B illustrate a fixation device with a leaf spring.
Figure 10 illustrates a close-up of a portion of another fixation device.
Figure 11A illustrates a close-up of a portion of another fixation device.
Figures 11B and 11C each illustrate a close-up cross-sectional side view of a portion of another fixation device.
Figures 11D and 11E each illustrate a close-up cross-sectional transverse view of a portion of another fixation device.
Figure 12 illustrates a close-up of a portion of another fixation device.

### DETAILED DESCRIPTION

### I. INTRODUCTION

### A. Cardiac Physiology

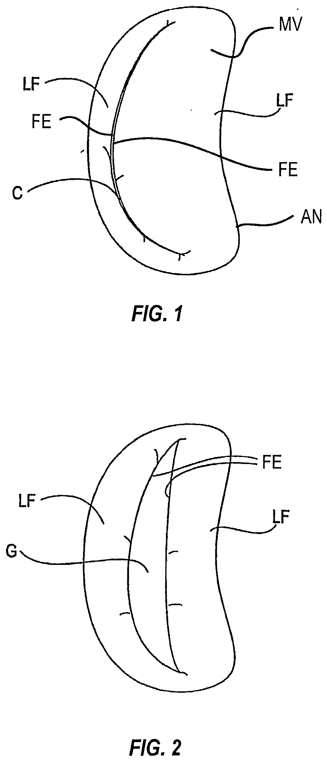

As shown in Figure 1, the mitral valve (MV) comprises a pair of leaflets (LF) having free edges (FE) which, in patients with normal heart structure and function, meet evenly to close along a line of coaptation (C). The leaflets (LF) attach to the surrounding heart structure along an annular region called the annulus (AN). The free edges (FE) of the leaflets (LF) are secured to the lower portions of the left ventricle LV through chordae tendinae (or "chordae").

As the left ventricle of a heart contracts (which is called "systole"), blood flow from the left ventricle to the left atrium through the mitral valve (MV) (called "mitral regurgitation") is usually prevented by the mitral valve.

Regurgitation occurs when the valve leaflets do not close properly and allow leakage from the left ventricle into the left atrium. A number of heart structural defects can cause mitral regurgitation. Figure 2 shows a mitral valve with a defect causing regurgitation through a gap (G).

### II. GENERAL OVERVIEW OF MITRAL VALVE FIXATION TECHNOLOGY

Several methods for repairing or replacing a defective mitral valve exist. Some defects in the mitral valve can be treated through intravascular procedures, where interventional tools and devices are introduced and removed from the heart through the blood vessels. One method of repairing certain mitral valve defects includes intravascular delivery of a fixation device to hold portions of the mitral valve tissues in a certain position. One or more interventional catheters may be used to deliver a fixation device to the mitral valve and install it there as an implant to treat mitral regurgitation.

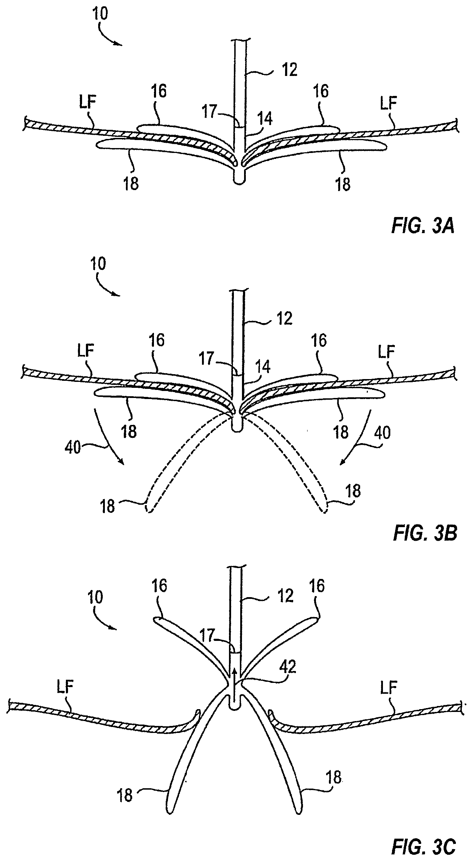

Figure 3A illustrates a schematic of an interventional tool 10 with a delivery shaft 12 and a fixation device 14. The tool 10 has approached the mitral valve MV from the atrial side and grasped the leaflets LF.

The fixation device 14 is releasably attached to the shaft 12 of the interventional tool 10 at the distal end of the shaft 12. In this application, when describing devices, "proximal" means the direction toward the end of the device to be manipulated by the user outside the patient's body, and "distal" means the direction toward the working end of the device that is positioned at the treatment site and away from the user. When describing the mitral valve, proximal means the atrial side of the leaflets and distal means the ventricular side of the leaflets.

The fixation device 14 comprises proximal elements 16 and distal elements 18 which protrude radially outward and are positionable on opposite sides of the leaflets LF as shown so as to capture or retain the leaflets therebetween. The fixation device 14 is coupleable to the shaft 12 by a coupling mechanism 17.

Figure 3B illustrates that the distal elements 18 may be moved in the direction of arrows 40 to an inverted position. The proximal elements 16 may be raised as shown in Figure 3C. In the inverted position, the device 14 may be repositioned and then be reverted to a grasping position against the leaflets as in Figure 3A. Or, the fixation device 14 may be withdrawn (indicated by arrow 42) from the leaflets as shown in Figure 3C. Such inversion reduces trauma to the leaflets and minimizes any entanglement of the device with surrounding tissues.

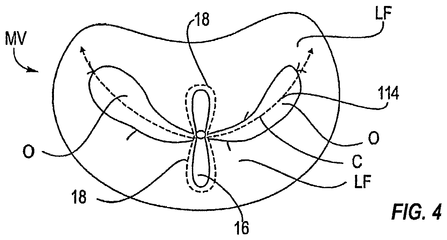

Figure 4 illustrates the fixation device 14 in a desired orientation in relation to the leaflets LF. The mitral valve MV is viewed from the atrial side, so the proximal elements 16 are shown in solid line and the distal elements 18 are shown in dashed line. The proximal and distal elements 16, 18 are positioned to be substantially perpendicular to the line of coaptation C. During diastole (when blood is flowing from the left atrium to the left ventricle), fixation device 14 holds the leaflets LF in position between the elements 16, 18 surrounded by openings or orifices O which result from the diastolic pressure gradient, as shown in Figure 4.

Once the leaflets are coapted in the desired arrangement, the fixation device 14 is detached from the shaft 12 and left behind as an implant.

### A. Exemplary Fixation Device

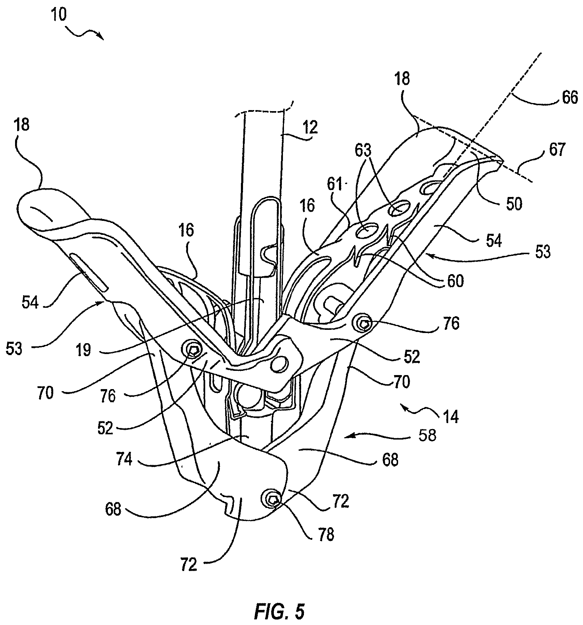

Figure 5 illustrates an exemplary fixation device 14. The fixation device 14 is shown coupled to a shaft 12 to form an interventional tool 10. The fixation device 14 includes a coupling member 19, a pair of opposed proximal elements 16, and a pair of opposed distal elements 18.

The distal elements 18 comprise elongate arms 53, each arm having a proximal end 52 rotatably connected to the coupling member 19 and a free end 54. Preferably, each free end 54 defines a curvature about two axes, axis 66 parallel to longitudinal axis of arms 53, and axis 67 perpendicular to axis 66 or the longitudinal axis of arms 53.

Arms 53 have engagement surfaces 50. Arms 53 and engagement surfaces 50 are configured to engage about 4-10 mm of tissue, and preferably about 6-8 mm along the longitudinal axis of arms 53. Arms 53 further include a plurality of openings.

The proximal elements 16 are preferably resiliently biased toward the distal elements 18. When the fixation device 14 is in the open position, each proximal element 16 is separated from the engagement surface 50 near the proximal end 52 of arm 53 and slopes toward the engagement surface 50 near the free end 54 with the free end of the proximal element 16 contacting engagement surface 50, as illustrated in Figure 5.

Proximal elements 16 include a plurality of openings 63 and scalloped side edges 61 to increase their grip on tissue. The proximal elements 16 optionally include a frictional element or multiple frictional elements to assist in grasping the leaflets. The frictional elements may comprise barbs 60 having tapering pointed tips extending toward engagement surfaces 50. Any suitable frictional elements may be used, such as prongs, windings, bands, barbs, grooves, channels, bumps, surface roughening, sintering, high-friction pads, coverings, coatings or a combination of these.

The proximal elements 16 may be covered with a fabric or other flexible material. Preferably, when fabrics or coverings are used in combination with barbs or other frictional features, such features will protrude through such fabric or other covering so as to contact any tissue engaged by proximal elements 16.

The fixation device 14 also includes an actuator or actuation mechanism 58. The actuation mechanism 58 comprises two link members or legs 68, each leg 68 having a first end 70 which is rotatably joined with one of the distal elements 18 at a riveted joint 76 and a second end 72 which is rotatably joined with a stud 74. The two legs 68 are each movably coupled to a base 69. Or, each leg 68 may be individually attached to the stud 74 by a separate rivet or pin. The stud 74 is joinable with an actuator rod which extends through the shaft 12 and is axially extendable and retractable to move the stud 74 and therefore the legs 68 which rotate the distal elements 18 between closed, open and inverted positions. Immobilization of the stud 74 holds the legs 68 in place and therefore holds the distal elements 18 in a desired position. The stud 74 may also be locked in place by a locking feature. This actuator rod and stud assembly may be considered a first means for selectively moving the distal elements between a first position in which the distal elements are in a collapsed, low profile configuration for delivery of the device, a second position in which the distal elements are in an expanded configuration for positioning the device relative to the mitral valve, and a third position in which the distal elements are secured in position against a portion of the leaflets adjacent the mitral valve on the ventricular side.

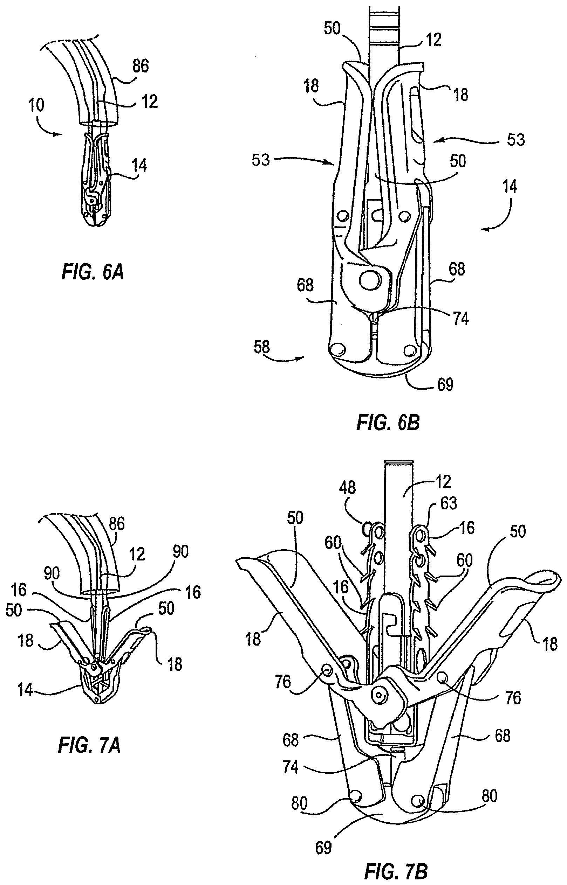

Figures 6A-6B, 7A-7B, and 8 illustrate various possible positions of the fixation device 14 of Figure 5. Figure 6A illustrates an interventional tool 10 delivered through a catheter 86. The catheter 86 may take the form of a guide catheter or sheath. The interventional tool 10 comprises a fixation device 14 coupled to a shaft 12 and the fixation device 14 is shown in the closed position.

Figure 6B illustrates a device similar to the device of Figure 6A in a larger view. In the closed position, the opposed pair of distal elements 18 are positioned so that the engagement surfaces 50 face each other. Each distal element 18 comprises an elongate arm 53 having a cupped or concave shape so that together the arms 53 surround the shaft 12. This provides a low profile for the fixation device 14.

Figures 7A-7B illustrate the fixation device 14 in the open position. In the open position, the distal elements 18 are rotated so that the engagement surfaces 50 face a first direction. Distal advancement of the actuator rod relative to shaft 12, and thus distal advancement of the stud 74 relative to coupling member 19, applies force to the distal elements 18 which begin to rotate around joints 76. Such rotation and movement of the distal elements 18 radially outward causes rotation of the legs 68 about joints 80 so that the legs 68 are directed slightly outwards. The stud 74 may be advanced to any desired distance correlating to a desired separation of the distal elements 18. In the open position, engagement surfaces 50 are disposed at an acute angle relative to shaft 12, and are preferably at an angle of between 90 and 180 degrees relative to each other. In the open position, the free ends 54 of arms 53 may have a span therebetween of about 10-20 mm, usually about 12-18 mm, and preferably about 14-16 mm.

Proximal elements 16 are typically biased outwardly toward arms 53. The proximal elements 16 may be moved inwardly toward the shaft 12 and held against the shaft 12 with the aid of proximal element lines 90 which can be in the form of sutures, wires, nitinol wire, rods, cables, polymeric lines, or other suitable structures. The proximal element lines 90 extend through the shaft 12 of the delivery catheter 86 and connect with the proximal elements 16. The proximal elements 16 are raised and lowered by manipulation of the proximal element lines 90. Once the device is properly positioned and deployed, the proximal element lines can be removed by withdrawing them through the catheter and out the proximal end of the device 10. The proximal element lines 90 may be considered a second means for selectively moving the proximal elements between a first position in which the proximal elements are in a collapsed, low profile configuration for delivery of the device and a second position in which the proximal elements are in an expanded configuration for engaging a portion of the leaflets adjacent the mitral valve on the atrial side.

In the open position, the fixation device 14 can engage the tissue which is to be approximated or treated. The interventional tool 10 is advanced through the mitral valve from the left atrium to the left ventricle. The distal elements 18 are then deployed by advancing actuator rod relative to shaft 12 to thereby reorient distal elements 18 to be perpendicular to the line of coaptation. The entire assembly is then withdrawn proximally and positioned so that the engagement surfaces 50 contact the ventricular surface of the valve leaflets, thereby engaging the left ventricle side surfaces of the leaflets. The proximal elements 16 remain on the atrial side of the valve leaflets so that the leaflets lie between the proximal and distal elements. The interventional tool 10 may be repeatedly manipulated to reposition the fixation device 14 so that the leaflets are properly contacted or grasped at a desired location. Repositioning is achieved with the fixation device in the open position. In some instances, regurgitation may also be checked while the device 14 is in the open position. If regurgitation is not satisfactorily reduced, the device may be repositioned and regurgitation checked again until the desired results are achieved.

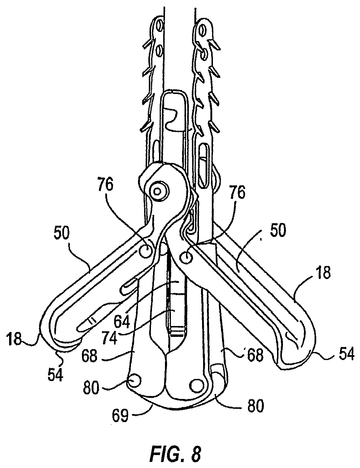

It may also be desired to invert distal elements 18 of the fixation device 14 to aid in repositioning or removal of the fixation device 14. Figure 8 illustrates the fixation device 14 in the inverted position. By further advancement of actuator rod relative to shaft 12, and thus stud 74 relative to coupling member 19, the distal elements 18 are further rotated so that the engagement surfaces 50 face outwardly and free ends 54 point distally, with each arm 53 forming an obtuse angle relative to shaft 12.

The angle between arms 53 when the device is inverted is preferably in the range of about 270 to 360 degrees. Further advancement of the stud 74 further rotates the distal elements 18 around joints 76. This rotation and movement of the distal elements 18 radially outward causes rotation of the legs 68 about joints 80 so that the legs 68 are returned toward their initial position, generally parallel to each other. The stud 74 may be advanced to any desired distance correlating to a desired inversion of the distal elements 18. Preferably, in the fully inverted position, the span between free ends 54 is no more than about 20 mm, usually less than about 16 mm, and preferably about 12-14 mm. Barbs 60 are angled slightly in the distal direction (away from the free ends of the proximal elements 16), reducing the risk that the barbs will catch on or lacerate tissue as the fixation device is withdrawn.

Once the distal elements 18 of the fixation device 14 have been positioned in a desired location against the left ventricle side surfaces of the valve leaflets, the leaflets may then be captured between the proximal elements 16 and the distal elements 18. The proximal elements 16 are lowered toward the engagement surfaces 50 by releasing tension from proximal element lines 90, thereby releasing proximal elements 16 so that they are then free to move, in response to the internal spring bias force formed into proximal elements 16, from a constrained, collapsed position to an expanded, deployed position and so that the leaflets are held between the proximal elements 16 and the distal elements 18. If regurgitation is not sufficiently reduced, the proximal elements 16 may be raised and the distal elements 18 adjusted or inverted to reposition the fixation device 14.

After the leaflets have been captured between the proximal and distal elements 16, 18 in a desired arrangement, the distal elements 18 may be locked to hold the leaflets LF in this position or the fixation device 14 may be returned to or toward a closed position. This is achieved by retraction of the stud 74 proximally relative to coupling member 19 so that the legs 68 of the actuation mechanism 58 apply an upwards force to the distal elements 18 which in turn rotate the distal elements 18 so that the engagement surfaces 50 again face one another. The released proximal elements 16 which are biased outwardly toward distal elements 18 are concurrently urged inwardly by the distal elements 18. The fixation device 14 may then be locked to hold the leaflets in this closed position. The fixation device 14 may then be released from the shaft 12.

The fixation device 14 optionally includes a locking mechanism for locking the device 14 in a particular position, such as an open, closed or inverted position or any position therebetween.

The locking mechanism may include a release harness. Applying tension to the release harness may unlock the locking mechanism.

The lock lines engage the release harnesses of the locking mechanism to lock and unlock the locking mechanism. The lock lines extend through the shaft of the delivery catheter. A handle attached to the proximal end of the shaft is used to manipulate and decouple the fixation device 14.

Additional disclosure regarding such fixation devices 14 may be found in PCT Publication No. WO 2004/103162 and U.S. Patent Publication No. 2015-0257883.

### B. Improved Grasping Mechanisms

Sometimes it can be difficult to capture or retain tissue within fixation device 14 so that fixation device 14 approximates or repairs the tissue as desired. Leaflet insertion may be assessed throughout the process of installing a fixation device 14, but it can be difficult to differentiate good and poor leaflet insertion and retention. For example, when fixation device 14 is used in endovascular or minimally invasive procedures, visualization of the capturing or retention of tissue may be difficult.

At times during the process of installing a fixation device 14, the tissue desired to be captured or retained between proximal elements 16 and distal elements 18 may seem to be securely captured or retained when it is actually only partially captured or insecurely captured. As a result, the free edges FE of leaflet tissue LF may later disassociate from the fixation device 14 and the fixation device 14 may then not properly coapt, approximate, or repair the tissue. Even if imaging methods make it possible to visualize when tissue is captured in the fixation device, they may not allow for a viewer to distinguish between securely and insecurely captured tissue. For example, color Doppler echo may show that regurgitation has been reduced, but it may not provide precise specifics on where along the leaflets LF fixation device 14 has captured the tissue, and whether the capturing is secure.

If a leaflet is poorly grasped between proximal elements 16 and distal elements 18, eventually that leaflet LF may separate from the fixation device 14. This may result in the fixation device 14 being attached to only one of the leaflets LF, or separating from both leaflets LF, and no longer functioning as desired.

In addition to difficulties arising from the imaging or visualization of the device 14 as it is installed, difficulty in capturing or retaining tissue within fixation device 14 may also result from the nature of tissue desired to be captured or retained. For example, when using fixation device 14 to fix mitral valve leaflets LF to each other to stop or reduce mitral valve regurgitation, the leaflets LF are constantly moving as the heart beats.

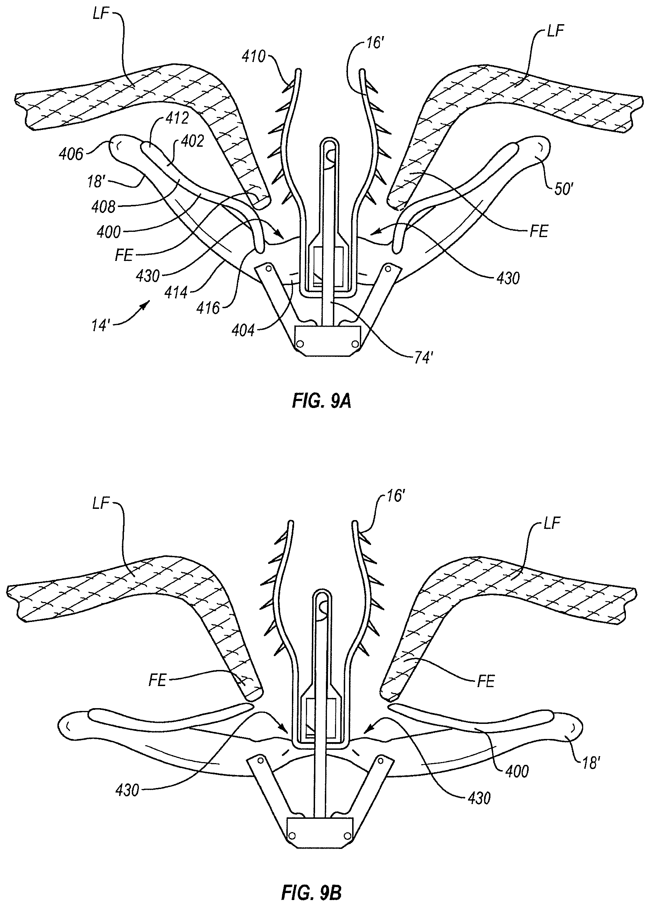

Figures 9 - 12 illustrate various examples that are intended to help a fixation device 14' capture and retain the free edges FE of leaflets LF during placement of the fixation device 14'. To do so, these examples include the addition of a retaining element 400 positioned on the proximal side of each distal element 18'. The retaining element 400 combines with frictional elements such as barbs 410 at the lower end of the proximal element 16' to capture the free edge FE of the leaflet upon its initial insertion and help retain it there until the proximal and distal elements are fully deployed. The lower end of the proximal element 16' is the end closest to the stud 74'.

The retaining element 400 and barbs 410 are configured to cooperate to allow the free edge FE of the leaflets LF to easily or freely move in a first direction toward the apex 430 formed between each proximal element 16' and the corresponding distal element 18', but at the same time to resist or prevent movement of the free edge FE of the leaflet tissue LF in the opposite direction away from apex 430. In this way, retaining element 400, in cooperation with the barbs 410, helps to retain the leaflets LF in the device 14' while the device is being positioned relative to the leaflets LF and before the proximal elements 16' and distal elements 18' are fully deployed.

The retaining element 400 may serve as a passive capture mechanism that retains leaflet tissue LF without needing to be activated. For example, the retaining element 400 may retain leaflet tissue LF in the device 14' when a length of tissue of about 4-10 mm, and preferably about 6-8 mm, is located along the longitudinal axis of the distal elements 18'. A retaining element 400 may be located on the distal elements 18', as shown in the illustrated examples, or the retaining element 400 may be located on the proximal elements 16', or it may be located on both the distal elements 18' and the proximal elements 16'. A retaining element 400 may hold a leaflet LF in place without closing the fixation element 18' and gripping element 16'.

As shown in Figures 9A-9B, in one example, a retaining element 400 may be a spring element 402 that retains leaflets LF inserted into the fixation device 14'. The spring element 402 may help capture or hold any leaflet LF that inserts past a given point along the distal elements 18' which is determined to be sufficient insertion depth.

Referring again to Figures 9A-B, the spring element 402 is on a distal element 18'. Each distal element 18' has a spring element 402 incorporated into or attached to the distal element 18'. The spring element 402 may be incorporated into or attached to the distal element 18' at a midpoint 414 between a first end 404 of the distal element 18' that attaches to a stud 74' and the free end 406 of the distal element 18'. It may also be incorporated into or attached to the distal element 18' closer to the free end 406 of each distal element 18' or to the first end 404 of each distal element 18'. As shown in Figures 9A-B, the fixed end 412 of the spring element 402 is located between the midpoint 414 and free end 406 of the distal element 18'. In addition, the spring element 402 of the retaining element 400 is elongate and can extend in an elongate fashion along substantially an entire length of the distal element 18' and associated distal engagement surface. Alternatively, the retaining element 400 can extend in an elongate fashion from a location near or adjacent first end 404 to a location distal a midpoint of the distal engagement surface of the distal element 18' or from a location near or adjacent second end 406 to a location distal a midpoint of the distal engagement surface of the distal element 18'.

The spring element 402 may comprise a low-force leaf spring 408 biased to push the spring element 402 towards the leaflet LF and encourage frictional elements or barbs 410 to be deeply inserted into the leaflet LF, so the leaflet LF remains in a fully seated state until distal elements 18' are further closed. As illustrated, barbs 410 are orientated at an angle pointing toward apex 430. With barbs 410 oriented in that direction, the leading edge LE of the leaflet tissue LF is allowed to move in a first direction toward apex 430 with little or no restriction or resistance. As the leading edge LE of the leaflet tissue moves toward apex 430, spring element 402 directs or urges the leaflet tissue LF toward and into contact with barbs 410. Once the leaflet tissue LF comes into contact with and engages the barbs 410, the angled orientation of the barbs 410 causes barbs 410 to penetrate into the leaflet tissue LF and then restricts or prevents movement of the leaflet tissue LF in the opposite direction away from apex 430. Thus, the combination of the retaining element 400 and the barbs 410 effectively function as a directional trap that permits the leaflet tissue LF to move in a first direction toward apex 430 with little or no resistance, while restricting or preventing movement of the leaflet tissue LF in a second or opposite direction away from apex 430.

The leaf spring 408 may have one or more lobes or a partial lobe. The leaf spring 408 may be biased to allow for little to no resistance to a leaflet LF as it inserts. It may have surface features, a pointed edge, or other elements that create resistance to make it difficult for the leaflet LF to retract out. Such surface features may include, for example, dimples, bumps, ridges, or indents.

For example, as shown in Figure 9A-B, the free end 416 of the spring element 402 may be configured to curve toward the distal elements 18' (as shown in Figure 9A) when tissue LF is being trapped, and curve away from the distal element 18' (as shown in Figure 9B) when tissue LF is being released. When the leaflet is entrapped between the distal elements 18' and the proximal elements 16', the free end 416 of the spring element 402 may be configured to lie flat against the distal element 18'.

The retaining element 400 helps the fixation device 14 capture tissue when proximal elements 16' are raised and distal elements 18' are still partially open. The retaining element 400 may be configured to urge the leaflet tissue against the barbs 410 on the proximal elements 16. The retaining element 400 may be a one-way mechanism that allows tissue to enter but not exit, such as a ratchet or something similar to a ratchet.

Because repositioning and regrasping the fixation device 14' is sometimes required, the one-way mechanism should have a way for the leaflet tissue LF to be permitted to escape. The retaining element can be designed to allow tissue to exit under certain circumstances, such as when the distal elements 18' are opened to approximately 180°, as shown in Figure 9B, or are opened even further in an inverted position, as shown in Figure 8. Or, when the proximal elements 16' are raised, the leaflet tissue LF may be released to allow regrasping.

To ensure that the leaflets LF are properly grasped in a fixation device 14', one method of using a device with a retaining element 400 such as a spring element 402 located near each set of distal elements 18' and proximal elements 16' is to first capture one or both leaflets LF in the spring element 402. The spring element urges the leaflets LF against the barbs 410 at the lower end of the proximal element 16' to capture the free edge FE of the leaflet LF upon its initial insertion and help retain it there until the proximal and distal elements 16, 18 are fully deployed.

It may be possible to confirm that one or more leaflets LF is captured based on imaging methods such as color Doppler echo. When leaflets LF are trapped between the spring element 402 and the barbs 410, then the proximal elements 16' may be lowered toward the surfaces 50' of the distal elements 18', so that the leaflets LF are held therebetween and the distal elements 18' may be locked to hold the leaflets LF in this position or the fixation device 14' may be returned to or toward a closed position.

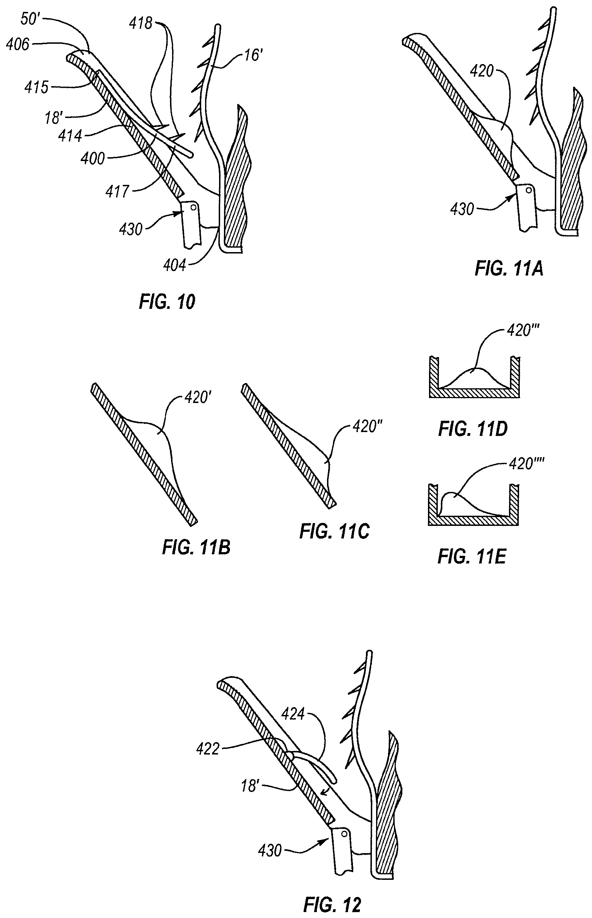

In another example, shown in Figure 10, a retaining element 400 comprises an arm 417. Each distal element 18' has an arm 417 incorporated into or attached to the distal element 18'. The arm 417 may be incorporated into or attached at a fixed end 415 to the distal element 18' at a midpoint 414 between a first end 404 of the distal element 18' that attaches to a stud 74' and the free end 406 of the distal element 18'. It may also be incorporated into or attached to the distal element 18' closer to the free end 406 of each distal element 18' or to the first end 404 of each distal element 18'. As shown in Figure 10, the fixed end 415 of the arm 417 is located between the midpoint 414 and free end 406 of the distal element 18'.

The arm 417 has a projection or projections 418 of a suitable shape and size to assist in retaining the leaflets LF in position. These projections 418 may have sharp tips located opposite to the arm 417, or sharp edges between their tips and the arm 417. They may comprise barbs having tapering pointed tips, scalloped edges, prongs, windings, bands, grooves, channels, bumps, surface roughening, sintering, high-friction pads, coverings, coatings or a combination of these. As shown in Figure 10, these projections may be oriented away from the surface 50' and angled away from the free ends 406 of the distal element 18'. They may also orient toward the free ends 406, or be perpendicular to the surface 50'. The projections may flex or collapse toward the distal element 18' when the fixation device 14' is closed and flex out to a fixed angle when the fixation device 14' is open. For example, the projections 418 may bias toward a fixed angle from the engagement surfaces 50', but may be pushed flat against the distal element 18' when the distal elements 18' close around the shaft 12.

The fixation device should be configured with enough space between the proximal elements 16' and the distal elements 18' for a leaflet LF to be easily inserted past the projections 418 on the distal elements 18'. The chordal tethered leaflets LF may be tensioned lightly upon the fixation device 14' just prior to closing the distal elements 18' and proximal elements 16'. They may also be securely affixed to the device 14' prior to closing the distal elements 18' and proximal elements 16'.

In one example, the arm 417 may be a flexible leaf-spring that pivots at a fixed end 415 and is positioned between the proximal element 16' and distal elements 18'. It may also include a system of projections 418 angled to allow entry of the tissue between the distal element 18' and proximal element 16', but to prevent retraction of the tissue LF. As shown, the projection 418 and leaf spring 417 may be combined in the same structure. In addition, the arm 417 of the retaining element 400 is elongate and can extend in an elongate fashion along substantially an entire length of the distal element 18' and associated distal engagement surface. Alternatively, the retaining element 400 can extend in an elongate fashion from a location near or adjacent first end 404 to a location distal a midpoint of the distal engagement surface of the distal element 18' or from a location near or adjacent second end 406 to a location distal a midpoint of the distal engagement surface of the distal element 18'.

As shown in Figure 11, in another example the retaining element 400 may comprise one or more protrusions 420. One or more protrusions 420 may be positioned close to the hinge point of the distal elements 18' on the engagement surface 50'. When a leaflet LF is inserted past the protrusion 420, the protrusion 420 may reduce leaflet detachment upon deployment of the fixation device 14' by directing or urging the leaflet LF into contact with the gripping surfaces or barbs 410 located on the opposing proximal element 16'. The protruding feature 420 may be located near a midpoint 414 between a first end 404 of the distal element 18' that attaches to a stud 74' and the free end 406 of the distal element 18'. It may also be incorporated into or attached to the distal element 18' closer to the free end 406 of each distal element 18' or to the first end 404 of each distal element 18'. A protruding feature 420 may be a rigid piece of material that is affixed to the engagement surface 50' of distal elements 18' and may be atraumatic to aid with directing or urging the leaflet LF while causing minimal damage to the leaflet LF, such as not penetrating or puncturing the leaflet LF. The protruding feature may be comprised of any biocompatible material or materials, such as a polymer, nitinol, or other alloys, or bioabsorbable materials.

The protruding feature 420 of the distal elements 18' may passively engage the leaflet tissue LF when leaflet tissue LF is sufficiently inserted into the device 14'. Or, the protruding feature 420 may be configured to help engage leaflet tissue and secure it into position when the proximal elements 16' are lowered and also secure the leaflet tissue LF. The protruding feature 420 may help entrap tissue between the protruding feature 420 and the gripping surfaces of the proximal elements 16'. The feature 420 may urge the tissue LF against the barbs 410.

While the protruding feature 420 is illustrated as including a generally curved or domed outer surface, it will be understood that various other surface orientations are appropriate while maintaining the atraumatic nature and ability to aid with directing or urging the leaflet LF. For instance, as illustrated in Figs. 11B - 11E, the protruding feature 420 can have a curved surface that is symmetric or asymmetric in (i) a direction from first end 404 towards the second end 406. (ii) a direction cross-wise, transverse, or oblique to the direction from first end 404 towards the second end 406, or (iii) both. So the protruding feature 420 can be symmetric in at least one axis, at least two axes, or in all three axes. Alternatively, the protruding feature 420 can be asymmetric in at least one axis, at least two axes, or in all three axes.

In another example, shown in Figure 12, the retaining element 400 may comprise a hinge 422 that is attached to the surface 50' of the distal elements 18'. The hinge 422 connects to an arm 424 that can swing toward and away from the distal element 18'. As shown in Figure 12, the arm 424 may bias toward the first end 404 of each distal element 18'. The arm 424 may be capable of laying parallel to or flat against the surface 50' while being oriented toward the first end 404 of the distal element 18'. It also may be capable of laying parallel to or flat against the surface 50' while being oriented toward the free end 406 of the distal element 18', and therefore capable of rotating 180°. The hinge 422 may restrict the movement of the arm 424 so that it can, for example, only lie parallel to the surface 50' while being oriented toward the first end 404 of the distal element 18' and be rotated about 90°, so that the angle formed between the arm 424 and the portion of the distal element 18' below the hinge 422 can be no greater than 90°. The hinge 422 may also be a pivoting element.

There may also be multiple arms 424 on the distal element 18'. For example, there may be two arms, each located the same distance between the ends 404 and 406, and positioned next to each other on the engagement surface 50'. If there are multiple retaining elements 400, such as multiple arms 424 or multiple spring elements 402, they may be configured to be positioned on either side of the barbs 410 on the proximal element. Retaining element or elements 400 may also be positioned to be located between barbs 410 on the proximal element 16', if there are multiple barbs 410 on the proximal element 16'. In addition, the arm 424 of the retaining element 400 is elongate and can extend in an elongate fashion along substantially an entire length of the distal element 18' and associated distal engagement surface. Alternatively, the retaining element 400, and associated arm 424, can extend in an elongate fashion from a location near or adjacent first end 404 to a location distal a midpoint of the distal engagement surface of the distal element 18' or from a location near or adjacent second end 406 to a location distal a midpoint of the distal engagement surface of the distal element 18'.

The distal elements 18 may be covered with a fabric or other flexible material. Preferably, when fabrics or coverings are used in combination with projections 418, such features will protrude through such fabric or other covering so as to contact the leaflet tissue LF.

Analogous to a mechanical pawl, the bias, angle, and direction of a retaining element 400 may allow the leaflet to fall or slide deeper towards the stud 74' without much resistance but may restrict the ability of the leaflet LF to move back out. By permitting the leaflet LF to easily enter but not permitting it to easily be removed from the fixation device 14', this may help entrap the leaflet LF in a fully inserted state.

In the examples described above, the retaining element 400 is a passive element. However, retaining element 400 may also include an active element such that, when a piece of leaflet tissue LF proceeds beyond or next to a portion of the retaining element 400, the retaining element 400 may automatically spring or deploy in such a way as to retain tissue LF in place.

In another example, a fixation device 14 or 14' may comprise a mechanical or physical sensor or some visual indicator of when a leaflet is properly inserted into the device prior to closing the distal elements 18' and deployment of the fixation device 14 or 14'. For example, a tactile sensor may be embedded near the first end 404 of each distal element. Each tactile sensor may provide a signal or indication when the leaflet LF touches the sensor, and the sensor may be located so that the leaflet LF will be unable or unlikely to touch the sensor unless the leaflet is adequately captured.

Yet another mechanism for enhancing the placement and retention of the leaflet tissue LF in the fixation device 14 or 14' is to facilitate actuation of each proximal element 16 or 16' and each distal element 18 or 18' independent from one another. When the proximal elements 16 or 16' for both leaflets LF are activated simultaneously, and the distal elements 18 or 18' for each leaflet LF are also activated simultaneously, it can be hard to capture both leaflets, because it is necessary to capture both at the same time. In other words, when the activation of both proximal elements 16 or 16' is symmetric, and the activation of both distal elements 18 or 18' is symmetric, the fixation device 14 or 14' is not able to grasp one leaflet first and then the other. If a catheter or the fixation device 14 or 14' is not properly positioned, or if either leaflet LF has redundant or loose length, the fixation device 14 or 14' may not fully seat the leaflets between each distal fixation element 18 or 18' and proximal gripping element 16 or 16'.

In one example, each proximal element 16 or 16' and/or each distal element 18 or 18' may be activated independently from each other. For example, there may be a separate proximal element line for each proximal element 16 or 16'. Similarly, there may be two actuator rods 64 which extend through the shaft 12, each of which may be configured to activate one distal element18 or 18'.

In addition to being used to repair mitral valves, these devices can be used in a variety of therapeutic procedures, including endovascular, minimally-invasive, and open surgical procedures, and can be used in various anatomical regions, including abdominal, thoracic, cardiovascular, intestinal, digestive, respiratory, and urinary systems, and other systems and tissues. The invention provides devices that may more successfully approximate and repair tissue by improving the capture of tissue into the devices.

The present invention may be embodied in other specific forms without departing from its essential characteristics. The described examples are to be considered in all respects only as illustrative and not restrictive. The scope of the invention is, therefore, indicated by the appended claims rather than by the foregoing description.

## Claims

1. A device (14') configured for intravascular delivery and for use in treating mitral valve defects in human patients, the device (14') comprising:
a body;
a pair of proximal elements (16'), each being coupled at a first end to the body on opposite sides of the body, each having a free second end, and each having a proximal engagement surface between the first and second ends thereof, the proximal engagement surface of each proximal element (16') comprising a frictional element (410); and
a pair of distal elements (18'), each being pivotally coupled at a first end (404) to the body on opposite sides of the body, each having a free second end (406), and each having a distal engagement surface between the first and second ends thereof (404, 406);
a first one of the proximal elements (16') cooperating with a first one of the distal elements (18') to form a space for receiving a portion of tissue therebetween, and a second one of the proximal elements (16') cooperating with a second one of the distal elements (18') to form a space for receiving a portion of the tissue therebetween, each said space having an open end and a closed end, the closed end forming an apex (430), wherein the frictional elements (410) comprise barbs (410) orientated at an angle pointing toward the apex (430); **characterized in that**
each distal element (18') further comprises a hinged or pivoting arm (424) positioned along the distal engagement surface and extending from a proximal end of the distal engagement surface to a location adjacent a distal end of the distal engagement surface, each hinged or pivoting arm (424) configured to cooperate with the corresponding frictional element (410) to capture a free edge of the tissue as the device is positioned relative to the mitral valve such that the combination of the hinged or pivoting arm (424) and the barbs (410) is configured to function as a directional trap that permits leaflet tissue to move in a first direction toward the apex (430) with little or no resistance, while restricting or preventing movement of the leaflet tissue in a second or opposite direction away from the apex (430).

2. The device (14') of claim 1, wherein each proximal engagement surface is configured to approximate and engage a portion of the leaflets adjacent the mitral valve on the atrial side.

3. The device (14') of claim 1 or claim 2, wherein the barbs (410) are configured to penetrate into the tissue.

4. The device (14') of claim 2, wherein each distal engagement surface is configured to approximate and engage a portion of the leaflets adjacent the mitral valve on the ventricular side.

5. The device (14') of claim 1 or claim 4, further comprising an actuator for selectively moving the distal elements (18') between a first position in which the distal elements (18') are in a collapsed, low profile configuration for delivery of the device (14'), a second position in which the distal elements (18') are in an expanded configuration for positioning the device (14') relative to the tissue, and a third position in which the distal elements (18') are secured in position against a portion of the tissue, and for selectively moving the proximal elements (16') between a first position in which the proximal elements (16') are in a collapsed, low profile configuration for delivery of the device (14') and a second position in which the proximal elements (16') are in an expanded configuration for engaging a portion of the tissue.

6. The device (14') of claim 1, wherein each of the distal elements (18') comprises a hinge or pivoting element (422) that is attached to the surface (50') of the distal element (18'), each hinge or pivoting element (422) connecting to the hinged or pivoting arm (424) that can swing toward and away from the respective distal element (18').

7. The device (14') of claim 6, wherein each arm (424) is capable of laying parallel to or flat against the surface (50') while being oriented toward the first end (404) of the respective distal element (18').

8. The device (14') of claim 7, wherein each arm (424) is capable of rotating 180°.

9. The device (14') of claim 1, wherein the hinged or pivoting arm (424) is elongate and extends in one of:
(a) an elongate fashion along substantially an entire length of the distal element (18') and associated distal engagement surface; and
(b) an elongate fashion from a location near or adjacent the free end (406) to a location distal a midpoint of the distal engagement surface of the distal element (18').

10. The device (14') of claim 1, wherein the distal elements (18') are covered with a fabric or other flexible material.

11. The device (14') of claim 5, wherein the actuator comprises a separate proximal element line for each proximal element (16').

12. The device (14') of claim 1, wherein the proximal elements (16') are resiliently biased toward the distal elements (18').

13. The device (14') of claim 1, wherein, each distal element (18') comprises an elongate arm having a cupped or concave shape.

14. The device (14') of claim 1, wherein the proximal elements (16') are raised and lowered by manipulation of proximal element lines, the proximal element lines being in a form selected from the list comprising sutures, wires, nitinol wire, rods, cables, and polymeric lines.

## Patentansprüche

1. Vorrichtung (14'), die für die intravaskuläre Zuführung und zur Behandlung von Mitralklappenfehlern bei menschlichen Patienten ausgelegt ist, wobei die Vorrichtung (14') umfasst:
einen Körper;
ein Paar proximaler Elemente (16'), von denen jedes an einem ersten Ende mit dem Körper auf gegenüberliegenden Seiten des Körpers verbunden ist, jedes ein freies zweites Ende aufweist und jedes zwischen seinem ersten und zweiten Ende eine proximale Eingriffsfläche aufweist, wobei die proximale Eingriffsfläche jedes proximalen Elements (16') ein Reibungselement (410) umfasst; sowie
ein Paar distaler Elemente (18'), von denen jedes an einem ersten Ende (404) schwenkbar mit dem Körper auf gegenüberliegenden Seiten des Körpers verbunden ist, jedes ein freies zweites Ende (406) aufweist und jedes zwischen seinem ersten und zweiten Ende (404, 406) eine distale Eingriffsfläche aufweist;
wobei ein erstes der proximalen Elemente (16') mit einem ersten der distalen Elemente (18') zusammenwirkt, um dazwischen einen Raum zur Aufnahme eines Gewebeteils zu bilden, und ein zweites der proximalen Elemente (16') mit einem zweiten der distalen Elemente (18') zusammenwirkt, um dazwischen einen Raum zur Aufnahme eines Gewebeteils zu bilden, wobei jeder dieser Räume ein offenes Ende und ein geschlossenes Ende aufweist, wobei das geschlossene Ende eine Spitze (430) bildet, wobei die Reibungselemente (410) Widerhaken (410) umfassen, die in einem Winkel ausgerichtet sind, der auf die Spitze (430) zeigt;
**dadurch gekennzeichnet, dass**
jedes distale Element (18') ferner einen schwenkbaren Arm (424) umfasst, der entlang der distalen Eingriffsfläche angeordnet ist und sich von einem proximalen Ende der distalen Eingriffsfläche bis zu einer zu einem distalen Ende der distalen Eingriffsfläche benachbarten Stelle erstreckt, wobei jeder schwenkbare Arm (424) so ausgebildet ist, dass er mit dem entsprechenden Reibungselement (410) zusammenwirkt, um eine freie Kante des Gewebes zu erfassen, wenn die Vorrichtung relativ zur Mitralklappe positioniert wird, derart, dass die Kombination aus dem schwenkbaren Arm (424) und den Widerhaken (410) so ausgebildet ist, dass sie als Richtungsfalle fungiert, die es dem Gewebe ermöglicht, sich in einer ersten Richtung zur Spitze (430) mit geringem oder gar keinem Widerstand zu bewegen, während die Bewegung des Gewebes in einer zweiten oder entgegengesetzten Richtung weg von der Spitze (430) eingeschränkt oder verhindert wird.

2. Vorrichtung (14') nach Anspruch 1, bei der jede proximale Eingriffsfläche konfiguriert ist, um sich einem Abschnitt der Segel, der an die Mitralklappe auf der Vorhofseite angrenzt, zu nähern und mit diesem in Eingriff zu kommen.

3. Vorrichtung (14') nach Anspruch 1 oder Anspruch 2, bei der die Widerhaken (410) konfiguriert ist, um in das Gewebe einzudringen.

4. Vorrichtung (14') nach Anspruch 2, bei der jede distale Eingriffsfläche konfiguriert ist, um sich einem Abschnitt der Segel, der an die Mitralklappe auf der Herzkammerseite angrenzt, zu nähern und mit diesem in Eingriff zu kommen.

5. Vorrichtung (14') nach Anspruch 1 oder Anspruch 4, die ferner einen Aktuator umfasst zum selektiven Bewegen der distalen Elemente (18') zwischen einer ersten Position, in der sich die distalen Elemente (18') zum Einführen der Vorrichtung (14') in einer zusammengeklappten Konfiguration mit geringem Profil befinden, einer zweiten Position, in der sich die distalen Elemente (18') zum Positionieren der Vorrichtung (14') relativ zum Gewebe in einer expandierten Konfiguration befinden, und einer dritten Position, in der die distalen Elemente (18') an einem Abschnitt des Gewebes fixiert sind, sowie zum selektiven Bewegen der proximalen Elemente (16') zwischen einer ersten Position, in der sich die proximalen Elemente (16') zum Einführen der Vorrichtung (14') in einer zusammengeklappten Konfiguration befinden, und einer zweiten Position, in der sich die proximalen Elemente (16') zum Eingreifen in einen Gewebebereich in einer expandierten Konfiguration befinden.

6. Vorrichtung (14') nach Anspruch 1, bei der jedes der distalen Elemente (18') ein Scharnier- oder Schwenkelement (422) umfasst, das an der Oberfläche (50') des distalen Elements (18') befestigt ist, wobei jedes Scharnier- oder Schwenkelement (422) mit dem schwenkbaren Arm (424) verbunden ist, der zum jeweiligen distalen Element (18) hin und von diesem weg schwenkbar ist.

7. Vorrichtung (14') nach Anspruch 6, bei der jeder Arm (424) in der Lage ist, parallel zur Oberfläche (50') oder flach an dieser anliegend zu verlaufen, während er auf das erste Ende (404) des jeweiligen distalen Elements (18') ausgerichtet ist.

8. Vorrichtung (14') nach Anspruch 7, bei der jeder Arm (424) um 180° drehbar ist.

9. Vorrichtung (14') nach Anspruch 1, bei der der schwenkbare Arm (424) langgestreckt ist und sich entweder
(a) im Längsverlauf im Wesentlichen entlang der gesamten Länge des distalen Elements (18') und der zugehörigen distalen Eingriffsfläche; oder
(b) im Längsverlauf von einer Stelle nahe oder neben dem freien Ende (406) bis zu einer Stelle distal eines Mittelpunkts der distalen Eingriffsfläche des distalen Elements (18') erstreckt.

10. Vorrichtung (14') nach Anspruch 1, bei der die distalen Elemente (18') mit einem Gewebe oder einem anderen flexiblen Material überzogen sind.

11. Vorrichtung (14') nach Anspruch 5, bei der der Aktuator für jedes proximale Element (16') eine separate proximale Elementleine umfasst.

12. Vorrichtung (14') nach Anspruch 1, bei der die proximalen Elemente (16') elastisch in Richtung der distalen Elemente (18') vorgespannt sind.

13. Vorrichtung (14') nach Anspruch 1, bei der jedes distale Element (18') einen langgestreckten Arm von schalenförmiger oder konkaver Gestalt umfasst.

14. Vorrichtung (14') nach Anspruch 1, bei der die proximalen Elemente (16') durch Betätigung von proximalen Elementleinen angehoben und abgesenkt werden, wobei die proximalen Elementleinen von einer Form sind, die aus der Liste ausgewählt ist, die Nahtmaterial, Drähte, Nitinoldraht, Stäbe, Kabel und Polymerleinen umfasst.

## Revendications

1. Dispositif (14') configuré pour une administration intravasculaire et destiné à être utilisé dans le traitement de défauts de la valve mitrale chez des patients humains, le dispositif (14') comprenant :
un corps ;
une paire d'éléments proximaux (16'), chacun étant accouplé au niveau d'une première extrémité au corps sur des côtés opposés du corps, chacun présentant une seconde extrémité libre, et chacun présentant une surface de mise en prise proximale entre les première et seconde extrémités de celui-ci, la surface de mise en prise proximale de chaque élément proximal (16') comprenant un élément de friction (410) ; et
une paire d'éléments distaux (18'), chacun étant accouplé de manière pivotante au niveau d'une première extrémité (404) au corps sur des côtés opposés du corps, chacun présentant une seconde extrémité libre (406), et chacun présentant une surface de mise en prise distale entre les première et seconde extrémités de celui-ci (404, 406) ;
un premier des éléments proximaux (16') coopérant avec un premier des éléments distaux (18') pour former un espace permettant de recevoir une partie de tissu entre eux, et un second des éléments proximaux (16') coopérant avec un second des éléments distaux (18') pour former un espace permettant de recevoir une partie du tissu entre eux, chaque dit espace présentant une extrémité ouverte et une extrémité fermée, l'extrémité fermée formant un sommet (430), dans lequel les éléments de friction (410) comprennent des barbes (410) orientées selon un angle dirigé vers le sommet (430) ; **caractérisé en ce que**
chaque élément distal (18') comprend en outre un bras articulé ou pivotant (424) positionné le long de la surface de mise en prise distale et s'étendant d'une extrémité proximale de la surface de mise en prise distale à un emplacement adjacent à une extrémité distale de la surface de mise en prise distale, chaque bras articulé ou pivotant (424) étant configuré pour coopérer avec l'élément de friction correspondant (410) pour capturer un bord libre du tissu lorsque le dispositif est positionné par rapport à la valve mitrale de sorte que la combinaison du bras articulé ou pivotant (424) et des barbes (410) soit configurée pour fonctionner comme un piège directionnel qui permet au tissu de valvule de se déplacer dans une première direction vers le sommet (430) avec peu ou pas de résistance, tout en limitant ou en empêchant le mouvement du tissu de valvule dans une seconde direction ou une direction opposée s'éloignant du sommet (430).

2. Dispositif (14') selon la revendication 1, dans lequel chaque surface de mise en prise proximale est configurée pour approcher et mettre en prise une partie des valvules adjacente à la valve mitrale sur le côté auriculaire.

3. Dispositif (14') selon la revendication 1 ou la revendication 2, dans lequel les barbes (410) sont configurées pour pénétrer dans le tissu.

4. Dispositif (14') selon la revendication 2, dans lequel chaque surface de mise en prise distale est configurée pour approcher et mettre en prise une partie des valvules adjacente à la valve mitrale sur le côté ventriculaire.

5. Dispositif (14') selon la revendication 1 ou la revendication 4, comprenant en outre un actionneur permettant de déplacer sélectivement les éléments distaux (18') entre une première position dans laquelle les éléments distaux (18') sont dans une configuration affaissée et à profil bas pour l'administration du dispositif (14'), une deuxième position dans laquelle les éléments distaux (18') sont dans une configuration déployée permettant de positionner le dispositif (14') par rapport au tissu, et une troisième position dans laquelle les éléments distaux (18') sont fixés en position contre une partie du tissu, et permettant de déplacer sélectivement les éléments proximaux (16') entre une première position dans laquelle les éléments proximaux (16') sont dans une configuration affaissée et à profil bas pour l'administration du dispositif (14') et une seconde position dans laquelle les éléments proximaux (16') sont dans une configuration déployée permettant de mettre en prise une partie du tissu.

6. Dispositif (14') selon la revendication 1, dans lequel chacun des éléments distaux (18') comprend une charnière ou un élément pivotant (422) qui est fixé(e) à la surface (50') de l'élément distal (18'), chaque charnière ou élément pivotant (422) se raccordant au bras articulé ou pivotant (424) qui peut osciller en s'éloignant et en se rapprochant de l'élément distal respectif (18').

7. Dispositif (14') selon la revendication 6, dans lequel chaque bras (424) est capable de reposer parallèlement à ou à plat contre la surface (50') tout en étant orienté vers la première extrémité (404) de l'élément distal respectif (18').

8. Dispositif (14') selon la revendication 7, dans lequel chaque bras (424) est capable de tourner à 180 °.

9. Dispositif (14') selon la revendication 1, dans lequel le bras articulé ou pivotant (424) est allongé et s'étend sous :
(a) une forme allongée sur sensiblement toute une longueur de l'élément distal (18') et de la surface de mise en prise distale associée ; ou
(b) une forme allongée à partir d'un emplacement proche de ou adjacent à l'extrémité libre (406) jusqu'à un emplacement distal à un point médian de la surface de mise en prise distale de l'élément distal (18').

10. Dispositif (14') selon la revendication 1, dans lequel les éléments distaux (18') sont recouverts d'un tissu ou d'un autre matériau flexible.

11. Dispositif (14') selon la revendication 5, dans lequel l'actionneur comprend une ligne d'élément proximal séparée pour chaque élément proximal (16').

12. Dispositif (14') selon la revendication 1, dans lequel les éléments proximaux (16') sont sollicités de manière élastique en direction des éléments distaux (18').

13. Dispositif (14') selon la revendication 1, dans lequel chaque élément distal (18') comprend un bras allongé présentant une forme en coupe ou concave.

14. Dispositif (14') selon la revendication 1, dans lequel les éléments proximaux (16') sont soulevés et abaissés par manipulation de lignes d'éléments proximaux, les lignes d'éléments proximaux étant sous une forme sélectionnée dans la liste comprenant des sutures, des fils, des fils de nitinol, des tiges, des câbles et des lignes polymères.
